(19)

Europäisches
Patentamt
European
Patent Office
Office européen
des brevets

(11) **EP 4 385 495 A1**

(12) **EUROPEAN PATENT APPLICATION**

(43) Date of publication:
**19.06.2024 Bulletin 2024/25**

(21) Application number: **22386094.1**

(22) Date of filing: **15.12.2022**

(51) International Patent Classification (IPC):
**A61K 9/00** *(2006.01)* **A61K 9/51** *(2006.01)*

(52) Cooperative Patent Classification (CPC):
**A61K 9/5153; A61K 9/0019; A61K 9/5161**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicants:
- **National Technical University of Athens**
  **10682 Athens (GR)**
- **Democritus University of Thrace**
  **69100 Komotini (GR)**
- **Theracell Advanced Biotechnology S.A.**
  **14564 Kifissia (GR)**

(72) Inventors:
- **Detsi, Anastasia**
  **15780 Athens (GR)**
- **Kavetsou, Eleni**
  **15780 Athens (GR)**
- **Tzani, Andromachi**
  **15780 Athens (GR)**

- **Kostopoulou, Ioanna**
  **15780 Athens (GR)**
- **Pitterou, Ioanna**
  **15780 Athens (GR)**
- **Zoumpoulakis, Panagiotis**
  **15780 Athens (GR)**
- **Kontogiorgis, Christos**
  **68100 Alexandroupolis (GR)**
- **Deligiannidou, Eirini**
  **68100 Alexandroupolis (GR)**
- **Iliou, Triantafylia**
  **68100 Alexandroupolis (GR)**
- **Sapanidou, Christina**
  **68100 Alexandroupolis (GR)**
- **Fatouros, Polychronis**
  **14564 Kifissia (GR)**
- **Kontou, Maria**
  **14564 Kifissia (GR)**
- **Antoniou, Athanasia**
  **14564 Kifissia (GR)**

(74) Representative: **Roukounas, Dimitrios**
  **c/o Regus Business Center**
  **Nymphenburger Straße 4**
  **80335 München (DE)**

(54) **NANOSYSTEMS FOR THE ENCAPSULATION OF THERAPEUTIC AGENTS**

(57)     A nanoparticle comprising a therapeutic agent exhibiting anti-inflammatory or analgesic activity encapsulated in polylactic acid or poly (lactic-co-glycolic) acid, wherein the nanoparticle is coated with chitosan.

EP 4 385 495 A1

**Description**

**FIELD OF THE INVENTION**

The present invention relates to nanosystems encapsulating a therapeutic agent having anti-inflammatory or analgisic activity in biocompatible, biodegradable and nontoxic carriers.

**BACKGROUND OF THE INVENTION**

Osteoarthritis (OA), the most common type of arthritis, is a chronic, degenerative, highly aggravating and progressively developing disease of musculoskeletal system. OA is characterized by wear and tear of articular cartilage and reactive growth of new bone in the form of ostephytes covering the surface of the bones within the joint, as well as from bone hyperplasia in the boundaries of the articular surfaces of the bones within the joint. The synovial membrane reacts by creating more synovial fluid resulting in edema. In some cases, the lesions are so extensive that are able to cause deformation of the joint. (Ali Mobasheri, Mark Batt, An update on the pathophysiology of osteoarthritis, Annals of Physical and Rehabilitation Medicine, Volume 59, Issues 5-6, 2016, Pages 333-339, ISSN 1877-0657, https://doi.org/10.1016/j.rehab.2016.07.004).

Risk factors for osteoarthritis are various genetic factors mechanisms, obesity and recurrent occupational mechanical stress and strain. According to the Hellenic Rheumatological Research Foundation, 13.1% of Greeks over the age of 50 suffer from symptomatic OA while more than 32.5 million U.S. adults have osteoarthritis (CDC, 2020). Globally, more than 22% of adults around the world who are older than 40 have knee osteoarthritis. OA is one of the most expensive treatments when joint replacement surgery is required in the United States (United States Bone and Joint Initiative: The Burden of Musculoskeletal Diseases in the United States (BMUS), Fourth Edition. Rosemont, IL. Available at http://www.boneandjointburden.orgexternal icon. Accessed on February 25, 2020). Especially, OA was the second most costly health condition treated at US hospitals in 2013, meaning $16.5 billion, or 4.3%, of the combined costs for all hospitalizations. The estimated number of adults in the US with a documented diagnosis of arthritis, mainly with a diagnosis of OA, is projected to increase to approximately $67 \times 10^6$ by 2030.

Signaling pathways involved in the inflammatory response to osteoarthritis are reported (Lotz M, Martel-Pelletier J, Christiansen C, et al. Value of biomarkers in osteoarthritis: current status and perspectives [published correction appears in Ann Rheum Dis. 2017 May 25:]. Ann Rheum Dis. 2013;72(11):1756-1763. doi:10.1136/annrheumdis-2013-203726) to be the following:

1. The progression of OA alters the production and function of certain cytokines. In particular, (a) the pro-inflammatory interleukins IL-1$\beta$, IL-6, IL-15, IL-17, and IL-18, (b) the anti-inflammatory interleukins IL-4, IL-10, and IL-13, and (c) tumor necrosis factor $\alpha$ (TNF-$\alpha$).

2. The enzymes COX-1 and especially COX-2 are responsible for the biosynthesis of prostaglandin E2 (PGE2) which reduces pain levels. COX-2 production is induced by proinflammatory cytokines such as IL-1 as well as TNF-$\alpha$.

3. Matrix metalloproteinase-13 (MMP-13) is a major extracellular matrix catabolic enzyme which targets cartilage for degradation and is highly expressed in the joint of patients with osteoarthritis. Moreover, MMP-13 targets type II collagen in cartilage for degradation, as well as degrades proteoglycan, types IV and type IX collagen, osteonectin and perlecan in cartilage. In OA, the balance between anabolica and catabolic processes tilts predominantly towards the catabolic ones, with increased MMP-13 being a major driver of this disbalance.

4. Abnormal metabolism of chondrocytes leads to the production of a large concentration of free radicals that exceed the regulatory antioxidant capacity. Free radicals are important signaling molecules that enhance the inflammatory response. They can also induce structural and functional changes in the extracellular matrix leading to joint stiffness and fragility in patients with OA.

5. The enzyme 5-lipoxygenase (5-LOX) and the P2X7 receptor are generally involved in the development of inflammatory arthritis and rheumatoid arthritis.

The symptoms of OA are the breakdown of articular cartilage accompanied by inflammation (occurrence of swelling), tingling, stiffness, tenderness, deformities and pain in the joints. Symptoms such as pain, stiffness, and gelling in OA (An update on the pathophysiology of osteoarthritis, Annals of Physical and Rehabilitation Medicine, Volume 59, Issues 5-6, 2016, Pages 333-339, ISSN 1877-0657, hftps://doi.org/10.1016/j.rehab.2016.07.004) contribute to patients' functional limitations (loss of flexibility), with a well-documented association of pain level with the degree of functional limitation (Flores-Garza, P.P.; Garcia-Espinoza, Ó.A.; Salas-Longoria, K.; Salas-Fraire, Ó. Association between ischiotibial muscle flexibility, functional capacity and pain in patients with knee osteoarthritis. Med. Univ. 2017, 19, 111-114, doi:10.1016/j.rmu.2017.06.001; Arul Pragassame S; Mohandas Kurup VK; Soundarya N A comparative study on the effectiveness of PNF stretching versus static stretching on Pain and Hamstring flexibility in osteoarthritis knee patients.

Int. J. Res. Pharm. Sci. 2019, doi:10.26452/ijrps.v10i3.1312).

International guidelines (Hernández-Díaz, C.; van Schoor, N.; Khalil, A.A.F. Osteoarthritis. In Comorbidity in Rheumatic Diseases; Springer Science and Business Media LLC: Cham, Switzerland, 2017; pp. 197-206) for the management of OA are based in three core interventions: weight control, general and joint-specific exercise, and education, while cases unaffected by these key interventions are often managed through non-pharmacological and surgical approaches, in the context of distressing OA, along with the use of analgesia and that favorably modify joint biomechanics. Today, OA treatment rehabilitation is distinguished in surgery (eg arthroplasty) for very progressed disease, in stem cell therapy as well as in conservative treatment involving drug therapy, physiotherapy and change in the patient's lifestyle (weight loss) (Wallis, J.A.; Barton, C.J.; Brusco, N.K.; Kemp, J.L.; Sherwood, J.; Young, K.; Jennings, S.; Trivett, A.; Ackerman, I.N. Exploring views of orthopaedic surgeons, rheumatologists and general practitioners about osteoarthritis management. Musculoskeletal Care 2021, doi:10.1002/msc.1549; Jadidi, S. A Review of Non-Surgical Pain Management in Osteoarthritis. Cureus 2020, doi:10.7759/cureus.10829; Kan, H.S.; Chan, P.K.; Chiu, K.Y.; Yan, C.H.; Yeung, S.S.; Ng, Y.L.; Shiu, K.W.; Ho, T. Non-surgical treatment of knee osteoarthritis. Hong Kong Med. J. 2019; Nelson, A.E.; Allen, K.D.; Golightly, Y.M.; Goode, A.P.; Jordan, J.M. A systematic review of recommendations and guidelines for the management of osteoarthritis: The Chronic Osteoarthritis Management Initiative of the U.S. Bone and Joint Initiative. Semin. Arthritis Rheum. 2014, doi:10.1016/j.semarthrit.2013.11.012). Medication is symptomatic and includes administration of analgesics and Non-Steroids Anti-Inflammatory Drugs (NSAIDs) by preventing and treating the formation of inflammation. In cases where NSAID treatment is insufficient, intra-articular injectable corticosteroids are used for the treatment of symptoms, however the short duration of action forces patients to undergo repeated intra-articular injections. In addition, long-term use of NSAIDs may lead to reduced effectiveness (Crofford, L.J. Use of NSAIDs in treating patients with arthritis. Arthritis Res. Ther. 2013; Van de Laar, M. Pain Treatment in Arthritis-Related Pain: Beyond NSAIDs. Open Rheumatol. J. 2012, doi:10.2174/1874312901206010320). Furthermore, treatment is limited to relieving symptoms and is ineffective to improve the underlying condition. Even in the case of regenerative cell therapy (eg intra-articular injection of mesenchymal stromal cells), during the first period the patient receives additional treatment to reduce inflammation and pain.

Various active ingredients, including active ingredients derived from natural products (small organic molecules isolated from plants and microorganisms) have emerged as promising potential agents for the management of OA (Deligiannidou, G.E.; Papadopoulos, R.E.; Kontogiorgis, C.; Detsi, A.; Bezirtzoglou, E.; Constantinides, T. Unraveling natural products' role in osteoarthritis management-an overview. Antioxidants 2020). However, there are numerous challenges associated with the deployment of such an active ingredient in pharmaceutical compositions. For example, in order to fulfil their potential, the active ingredient should preferably be administered locally and therefore the composition should be in a form allowing local application. Furthermore, the composition should be able to achieve high loading and controlled release of the active ingredient. In addition, the composition should safeguard the stability of the active ingredient.

## SUMMARY OF THE INVENTION

The present invention provides a nanoparticle comprising a therapeutic agent exhibiting anti-inflammatory or analgesic activity encapsulated in polylactic acid (PLA) or poly (lactic-co-glycolic) acid (PLGA), wherein the nanoparticle is coated with chitosan.

The present invention provides also a pharmaceutical composition comprising a nanoparticle comprising a therapeutic agent exhibiting anti-inflammatory or analgesic activity encapsulated in PLA or PLGA, wherein the nanoparticle is coated with chitosan.

The present invention provides also the use of the nanoparticle or of the pharmaceutical composition of the present invention in the treatment of osteoarthritis.

## DETAILED DESCRIPTION OF THE INVENTION

The present inventors have found that a nanoparticle comprising a therapeutic agent encapsulated in an anionic biodegradable polymer, wherein the nanoparticle is coated with chitosan, exhibits excellent characteristics. Namely, it was found that a nanoparticle according to the present invention improves the properties of the therapeutic agent. These improvements include increased protection of the therapeutic agent, for example, from photodegradation, oxidation, or moisture, increased stability of the therapeutic agent, achievement of sustained release of the therapeutic agent, possibility of topical administration of the therapeutic agent, improvement of the therapeutic efficacy and the toxicological profile of the therapeutic agent, decrease of the required amount of the therapeutic agent to obtain the desired therapeutic effect.

Thus, the present invention provides a nanoparticle comprising a therapeutic agent exhibiting anti-inflammatory or analgesic activity encapsulated in polylactic acid (PLA) or poly (lactic-co-glycolic) acid (PLGA), wherein the nanoparticle is coated with chitosan.

Preferably, the therapeutic agent is a naturally occurring compound exhibiting anti-inflammatory or analgesic activity. Examples of therapeutic agents which can be used in the present invention include cannabidiol, cinnamaldehyde, sphaeralcic acid, shikimic acid, physalin A, avenanthramide C, polyphenols (such **as** hydroxytyrosol, curcumin, epigallocatechin-3-gallate, monomethyl pinosylvin, pinosylvin, resveratrol, punicalagin, psoralidin, rosmarinic acid), carotenoids (such as astaxanthin, crocin), flavonoids (such as alpinetin, apigenin, astragalin, baicalein, bavachin, biochanin A, casticin, chrysin, cynaroside, daidzein, delphinidin, engeletin, epimedin C, eupatilin, galangin, hyperoside, icariin, isorhamnetin, jaceosidin, kaempferol, luteolin, malvidin, morin, naringin, nepetin, pinocembrin, prunetin, puerarin, quercetin, silymarin, wogonin, cyanidin-3-O-glucoside, peonidin-3-O-glucoside), terpenes/terpenoids [such as Iridoid glycosides (such as aucubin, geniposide, monotropein, loganin), secoiridoid glycosides (such as oleuropein, gentiopicroside), triterpene saponins (such as ginsenosides), maslinic acid, oleanolic acid, betulin, kirenol, andrographolide, alpha-pinene, glaucocalyxin A], alkaloids (such as berberine, leonurine hydrochloride, matrine, piperine, sinomenine, tetrandrine, benzoylaconitine, benzoylhypaconitine),phenolic acids (such as ferulic acid, protocatechuic acid, vanillic acid, 4-hydroxybenzoic acid, gallic acid), quinone methides (such as celastrol), quinones (such as thymoquinone), coumarins (such as osthole, isofraxidin, scopoletin, tomentin, urolithin A), lignans (such as honokiol, schisantherin A, sesamin, phyllanthin , hypophyllanthin), alkylpyrazines (such as tetramethylpyrazines, for example ligustrazine), saponins (such as ecliptasaponin A, notoginsenoside R1), sapogenins (such as diosgenin). More preferably, the therapeutic agent is selected from naringin, cannabidiol, hydroxytyrosol, rosmarinic acid, baicalein, chrysin, quercetine, silymarin, cyanidin-3-O-glucoside, peonidin-3-O-glucoside. Even more preferably, the therapeutic agent is naringin.

Preferably, the PLA in the nanoparticle of the present invention has an average molecular weight from 2000g/mol to 150000g/mol. More preferably, the PLA has an average molecular weight from 10000g/mol to 70000g/mol. Even more preferably, the PLA has an average molecular weight from 30000g/mol to 60000g/mol. Most preferably, the PLA has an average molecular weight of 46000g/mol.

Poly(lactic-co-glycolic acid), also known as poly(D,L-lactic-co-glycolic acid), or PLGA, is a copolymer of polylactic acid (poly D,L lactic acid where D and L lactic acid forms are in equal ratio) and poly glycolic acid. Preferably, the PLGA in the nanoparticle of the present invention has a molar ratio of D,L-lactic acid to glycolic acid from 40:60 to 85:15. More preferably, the molar ratio of D,L-lactic acid to glycolic acid is from 45:55 to 75:25. Even more preferably, the molar ratio of D,L-lactic acid to glycolic acid is 50:50.

Preferably, the PLGA in the nanoparticle of the present invention has an average molecular weight from 5000g/mol to 75000g/mol. More preferably, the PLGA has an average molecular weight from 5000g/mol to 40000g/mol. Even more preferably, the PLGA has an average molecular weight 15000 g/mol.

The nanoparticle of the present invention is coated with chitosan. Preferably, the average molecular weight of chitosan is from 2000g/mol to 80000g/mol. More preferably, the average molecular weight of chitosan is from 5000 g/mol to 60000g/mol. Even more preferably, the average molecular weight of chitosan is 20000g/mol.

Preferably, the degree of deacetylation of chitosan is at least 80%, more preferably, at least 85% and even more preferably, at least 90%.

The nanoparticle according to the present invention has a diameter of 1000 nm or less. Preferably, the nanoparticle has a diameter from 150 nm to 600 nm. More preferably, the nanoparticle has a diameter from 190 nm to 550 nm.

Preferably, the content of PLA, or of PLGA in the nanoparticle of the present invention is from 20%w/w to 35%w/w. More preferably, the content of PLA, or of PLGA is from 22 %w/w to 31 %w/w.

Preferably, the content of the therapeutic agent in the nanoparticle of the present invention is from 1% w/w to 10%w/w. More preferably, the content of the therapeutic agent is from 3%w/w to 5%w/w.

Preferably, the content of chitosan in the nanoparticle of the present invention is from 55 %w/w to 75 %w/w. More preferably, the content of chitosan is from 65 %w/w to 75 %w/w.

Preferably, the mass ratio of PLA, or of PLGA to chitosan in the nanoparticle of the present invention is from 1:2 to 1:3. More preferably, the mass ratio of PLA, or of PLGA to chitosan is from 1:2.1 to 1:2.5. Even more preferably, the content of mass ratio of PLA, or of PLGA to chitosan is from 1:2.2 to 1:2.5.

The term "%w/w" used throughout the present application means grams of an ingredient per 100 grams of nanoparticles.

The nanoparticle of the present invention may further comprise an emulsifier, for example, when the encapsulation of the therapeutic agent in PLA or PLGA is carried out by the nanoprecipitation method, as disclosed hereinbelow. Examples of suitable emuslifiers include polymeric emulsifiers (such as poly(vinyl alcohol) - PVA, polyvinyl pyrrolidone/NaCl - PVP/ NaCl), non-ionic emulsifiers [such as a polysorbate [e.g. Tween® 20 (polyoxyethylenesorbitan monolaurate), Tween® 60 (Polyethylene glycol sorbitan monostearate), Tween® 65 (Polyoxyethylenesorbitan Tristearate), Tween® 80 (polyoxyethylenesorbitan monolaurate), Span® 80 (Sorbitane monooleate)], Vitamin E d-$\alpha$-tocopheryl polyethylene glycol 1000 succinate (TPGS1000)], amphiphilic emulsifiers ]such as poloxamers (such as Pluronic® F68, Pluronic® F127, Pluronic® F108, Pluronic® F68, Pluronic® F87)].

The nanoparticle of the present invention may further comprise a crosslinker. For example, when the coating of the nanoparticle with chitosan is carried out by the ionic gelation method, as disclosed hereinbelow, a crosslinker may be employed, which provided crosslinking of chitosan. Examples of suitable crosslinkers include sodium tripolyphosphate (TPP), pyrophosphate (PPi), sodium hexametaphosphate, sodium sulfate, dodecyl sulfate, dextran sulfate, k-carrageenan, i-carrageenan, exopolysaccharide B40, arabic gum, hyaluronic acid, mucic acid, sulfuric acid, trisodium citrate, citric acid, b-glycerophosphate, poly(aspartic acid) sodium salt and tannic acid.

A nanoparticle comprising the therapeutic agent encapsulated in PLA or PLGA can be prepared by following processes well known in the art. For example, it can be prepared by following the nanoprecipitation method. According to this method, an organic solution of PLA or PLGA is emulsified in an aqueous solution, with or without an emulsifier. Then the organic solvent is removed leading to nanoparticle formation.

Examples of emulsifiers which can be used in the nanoprecipitation method include polymeric emulsifiers (such as poly(vinyl alcohol) - PVA, polyvinyl pyrrolidone/NaCl - PVP/ NaCl), non-ionic emulsifiers [such as a polysorbate [e.g. Tween® 20 (polyoxyethylenesorbitan monolaurate), Tween® 60 (Polyethylene glycol sorbitan monostearate), Tween® 65 (Polyoxyethylenesorbitan Tristearate), Tween® 80 (polyoxyethylenesorbitan monolaurate), Span® 80 (Sorbitane monooleate)], Vitamin E d-α-tocopheryl polyethylene glycol 1000 succinate (TPGS1000)], amphiphilic emulsifiers ]such as poloxamers (such as Pluronic® F68, Pluronic® F127, Pluronic® F108, Pluronic® F68, Pluronic® F87)].

Examples of organic solvents that can be used in the nanoprecipitation method to dissolve PLA or PLGA and the therapeutic agent include, acetone, dichloromethane, ethyl acetate, dimethylsulfoxide, methanol and ethanol.

The nanoparticles of the present invention are coated with chitosan. Preferably, the coating of the nanoparticles is carried out by physical adsorption or by ionic gelation.

According to the physical adsorption method, chitosan is dissolved in an aqueous acidic solution and is then added to a dispersion of PLA or PLGA nanoparticles containing the encapsulated therapeutic agent. Examples of acids that can be used to dissolve chitosan include acetic, lactic, formic, L-ascorbic, L-glutamic, maleic, malic, succinic and citric acid.

According to the ionic gelation method, chitosan is dissolved in an aqueous acidic solution, the pH is adjusted preferably to 5.8 using a base, for example, NaOH solution, and the solution is then added to an aqueous dispersion of the PLA or PLGA nanoparticles containing the encapsulated therapeutic agent. The coating can be achieved by the addition of a physical crosslinker (a physical cross-linker provides cross-linking of the polymeric chains dissolved in aqueous solution). Examples of cross-linkers that can be used to achieve the coating of the nanoparticles include sodium tripolyphosphate (TPP), pyrophosphate (PPi), sodium hexametaphosphate, sodium sulfate, dodecyl sulfate, dextran sulfate, k-carrageenan, i-carrageenan, exopolysaccharide B40, arabic gum, hyaluronic acid, mucic acid, sulfuric acid, trisodium citrate, citric acid, b-glycerophosphate, poly(aspartic acid) sodium salt and tannic acid.

The present invention provides also a pharmaceutical composition comprising a nanoparticle as defined above combined with a pharmaceutically acceptable carrier. The composition can have different forms, well known in the art, such as solid or liquid forms, for example it can have the form of powder, solution, suspension, emulsion, gel, cream, ointment, spray, transdermal patch, or lotion. The pharmaceutically acceptable carrier may comprise one or more excipients, such as solvents, buffering agents, emulsifying agents, preservatives, antioxidants, diluents, binders and the like, which are well known in the art. Examples of solvents, include saline, water, phosphate buffered saline, ethanol, isopropyl alcohol and mixtures thereof. Examples of buffering agents include citric acid monohydrate, acetic acid, sodium acetate and sodium hydrogen phosphate. Examples of emulsifying agents include sodium lauryl sulfate, lecithin, polysorbate and sorbitan monooleate. Examples of preservatives include sodium benzoate, benzyl alcohol and para-hydroxy benzoic acids and their alkyl esters. Examples of antioxidants include sodium metabisulfite, butylated hydroxyanisole, butylated hydroxytoluene and ascorbic acid. Examples of diluents include lactose, sucrose, dextrose, mannitol and sorbitol. Examples of binders include hydroxypropylcellulose,' hydroxyethylcellulose, dextrose, xylitol, polyvinylpyrrolidone and polyethylene glycol. Preferably, the pharmaceutical composition is an injectable composition, such as a sterile injectable solution, suspension, or emulsion in a pharmaceutically acceptable solvent or suspending medium. Among the acceptable vehicles and solvents that may be employed are water, Ringer's solution and sodium chloride solution. In addition, oils, such as mono-, di- or triglycerides can be employed as a solvent or suspending medium. The injectable compositions can be sterilized, for example, by filtration through a bacteria-retaining filter.

The present invention provides also the use of a nanoparticle, as defined above, or a pharmaceutical composition, as defined above, in the treatment of osteoarthritis. Typically, the treatment involves the topical administration of a composition according to the present invention at, or near, the affected joint of a subject in need, preferably a human. The topical administration can be, for example, an injection, such as an intra-articular injection. The administered dosage and the frequency of administration depend on various factors, such as the therapeutic agent, the severity of the disease, or the form of the composition and can be determined by a person skilled in the art using common general knowledge.

**Examples**

The following examples illustrate the differences in the therapeutic and toxicologic activity of the flavonoid glycoside naringin when it is used in free (non-encapsulated) form, when it is encapsulated in PLA nanoparticles which do not comprise a coating and when it is encapsulated in nanoparticles according to the present invention. The sample codes used in the following examples are explained in Table 1 below.

Table 1: Sample codes used in the examples

| SAMPLE CODE | DESCRIPTION |
|---|---|
| S1 [NAR] | Naringin |
| S2 [PLA[NAR]] | Naringin encapsulated in PLA nanoparticles (Prepared as described in Example 1) |
| S3 CS[PLA[NAR]] | Naringin encapsulated in PLA nanoparticles coated with chitosan (prepared as described in Examples 1 and 2) |
| S4 CS[PLA[---]] | Blank PLA nanoparticles (they do not contain naringin) coated with chitosan (prepared as described in Examples 1 and 2) |

**Example 1: Encapsulation of naringin in PLA nanoparticles**

Naringin is encapsulated in poly(D,L-lactic acid) (PLA, Molecular Weight 46000 g/mol), using the nanoprecipitation method. In this method, the organic phase (oil phase) is first prepared, consisting of the solution of PLA in acetone and the solution of the substance to be encapsulated for drug load 40% (mass of the compound to be encapsulated per mass of PLA). The organic phase is then added to a continuous aqueous phase (oil-in-water emulsion) comprising 1% w/v poly (vinyl alcohol) (PVA, Molecular Weight 88,000-97,000 g/mol) followed by emulsification using ultrasound. The solvent is evaporated under reduced pressure, and the final dispersion of the formed nanoparticles is obtained by centrifugation at 19,000 rpm for 20 min. The supernatant is stored and is used for the determination of the inclusion efficiency. The nanoparticles are then washed by redispersion in deionized water twice using a centrifuge (19,000 rpm for 20 min). The purified nanoparticles are lyophilized and stored for further characterization.

**Example 2: Coating of PLA nanoparticles with chitosan**

Method A -Physical adsorption): The coating of the particles of Example 1 is achieved in an acidic environment by electrostatic deposition (or natural adsorption) of the positively charged chitosan on the negatively charged surface of PLA nanoparticles. Chitosan (Molecular Weight 20000 g/mol, degree of deacetylation >90%) is dissolved in 1% v / v aqueous acetic acid solution and added to a dispersion of PLA nanoparticles in mass proportion of Chitosan/PLA Nanoparticles = 2:1. The dispersion is allowed to stir for 24 hours and the resulting nanoparticles are collected and lyophilized for further characterization.
Method B - Ionic gelation method: Chitosan having Molecular Weight 20000 g/mol and degree of deacetylation >90%) (300 mg at a concentration of 0.3% w / v) is dissolved in 1% aqueous acetic acid solution and the pH is adjusted to 5.8 using a 20% w / v NaOH solution. Then, an aqueous dispersion of the PLA nanoparticles with encapsulated naringin ([PLA[NAR]]) of Example 1 is added to the chitosan solution (chitosan mass: [PLA[NAR]] mass = 2: 1)] followed by the addition of an aqueous solution of tripolyphosphoric acid (TPP) 0.1% w / w. With the addition of the TPP solution the formation of a turbid solution is observed, and the coating is achieved. Chitosan-coated PLA nanoparticles (CS[PLA[NAR]]) are lyophilized and taken up dry for further characterization.

**Example 3. In vitro release studies**

The release profile of naringin from **PLA[NAR]** and **CS[PLA[NAR]]** nanosystems, was studied at 37 °C using Dulbecco's Modified Eagle Medium/10% Fetal Bovine Serum (DMEM/10% FBS) as a medium, which is also used for human chondrocytes cell culture. For each experiment, specific amount of the nanosystems was added to the medium at a concentration of 4 mg/mL and the entire system was kept under continuous magnetic stirring (450 rpm) for approximately 12 days. At predetermined time intervals, 2 mL samples from the dispersion were withdrawn, and replaced with 2 mL of fresh medium in order to maintain a constant volume. The accumulated release amount of naringin from the nanosystems was determined using UV-vis spectroscopy. The release data revealed that in the case of **PLA[NAR]** nanosystem (Sample 2), a "burst effect" which is attributed to the release of the naringin adsorbed on the surface of the nanoparticles, occurs in 3 hours reaching 26%, followed by a slower, more constant release phase where 46% of naringin has been released in 12 days. Coating of PLA nanoparticles with chitosan **(CS[PLA[NAR]],** Sample 3) reduced the burst effect at 16% and the cumulative amount of NAR at 25% after 12 days (a biphasic release profile is also observed), indicating that as the layers increased, the naringin's release was prolonged. The sustained and prolonged release of naringin led to improvement of its bioavailability and biological activity as it was indicated in Examples 4-9. Chitosan coating improved the anti-inflammatory activity of the **PLA[NAR]** nanosystem.

### Example 4: Soybean lipoxygenase inhibition assay *in vitro*

The soybean lipoxygenase (LOX) test is a representative assay for anti-inflammatory activity since LOX is implicated in the inflammatory cascade. LOXs contain a "non-heme" iron per molecule in the catalytic active site as high-spin $Fe^{+2}$ in the native state and high-spin $Fe^{+3}$ in the activated state. LOX inhibition is following the ability of the compounds to reduce $Fe^{+3}$ at the active site to the catalytically inactive $Fe^{+2}$. The inhibitory activity of several LOX inhibitors, such as phenolic derivatives, can be attributed to their chelating capacity to $Fe^{+3}$. In brief, two reaction mixtures are prepared in UV cuvettes for each sample, the first reaction mixture contains LOX solution (dissolved in Tris buffer, pH 9) and the sample under examination, whereas the second reaction mixture also contained LLA solution (dissolved in 0.9% NaCl). Both reaction mixtures were measured at room temperature at 234 nm. The inhibition of LOX was measured in the presence of the same level of DMSO, which served as control. As positive control was used caffeic acid with IC50($\mu$M) = 600.

In this setting, the samples were evaluated in concentrations of 100 $\mu$M, 50 $\mu$M and 25 $\mu$M, exhibiting the following potential **(Table 2)**. Pure Naringin (sample S1) showed strong LOX inhibitory activitiy. Pure Naringin was evaluated at 48 and 72 h, but there was no significant difference between 24 h. In the 48h evaluation, the nanosystems (samples S2 and S3) showed the highest LOX inhibitory activity, which was also higher than that recorded for the pure compound S1. These results show that encapsulation preserves the bioactive potential of the pure compound and also allows it to exhibit inhibitory activity even in lower concentrations.

Table 2: Anti-inflammatory potential of the samples expressed as % inhibition of LOX

| Sample | Concentration | *24h* | *48h* | *72h* |
|---|---|---|---|---|
| **S1 [NAR]** | 25 $\mu$M | 7,1 | NA | NA |
| | 50 $\mu$M | 21,2 | NA | NA |
| | 100 $\mu$M | 44,6 | NA | ₁ NA |
| **S2 [PLA[NAR]]** | 25 $\mu$M | 2,6 | 0,8 | 0 |
| | 50 $\mu$M | 8,4 | 10,4 | 7,2 |
| | 100 $\mu$M | 19,4 | 53,2 | 17 |
| **S3 CS[PLA[NAR]]** | 25 $\mu$M | 20 | 53,5 | 24 |
| | 50 $\mu$M | 55 | 61 | 33 |
| | 100 $\mu$M | 48 | 93 | 46 |
| **S4 CS[PLA(---]]** | 25 $\mu$M | 0 | 0,2 | 0 |
| | 50 $\mu$M | 0 | 0,4 | 0 |
| | 100 $\mu$M | 0 | 8,5 | 1,9 |
| Note: Caffeic acid was used as positive control in this assay (IC50 = 600 $\mu$M). | | | | |

### Example 5: In vitro evaluation of the anti-inflammatory activity of the natural products and the corresponding nanosystems

#### a) IL-6 in RAW264.7 cells

The assay was carried using the MOUSE IL-6 ELISA KIT by ORIGENE_(CATALOG No. EA100128), which was based on the evaluation on Microwell Plates coated with antibody to mouse IL-6. The general protocol provided by the supplier for the evaluation of the samples as well as the development of the standard curve of IL-6 was followed. All reagents and working standards were prepared as directed in the instruction form and determined the number of microwell strips required to test the desired number of samples plus appropriate number of wells needed for running blanks and standards. Then 100$\mu$L of Standard, control, or sample, were added per well and the plate was cover with the adhesive strip provided and incubated for 1.5 hours at 37°C.

Afterwards, each well was aspirated and washed, and the process was repeated three times for a total of four washes by filling each well with Wash Buffer. After the fourth wash all washing buffer was removed and the plate was inverted and blotted against clean paper towels to remove any remaining fluid (this process will be further called as washing process). Then, the working solution of Biotin-Conjugate was added to each well and the plate was covered with a new adhesive strip and incubated for 1 hour at 37°C.

After incubation washing was carried out again, followed by the addition of the working solution of Streptavidin-HRP to each well. The plate was again covered with a new adhesive strip and incubated for 30 minutes at 37°C avoiding placing the plate in direct light for all further steps. The plate was again washed and then Substrate Solution was added to each well, and the plate was further incubated for 10-20 minutes at 37°C. Without any further washing Stop Solution were added to each well. Finally, the plate was gently tapped to ensure thorough mixing and determine the optical density (OD) of each well immediately, using a microplate reader set to 450 nm (optionally 630nm as the reference wavelength;610-650nm is acceptable).

The OD measured for all samples is reflected on the IL-6 standard curve which was developed following the same steps. The evaluation of IL-6 was carried out 48h after the dilution of nanosystems. This quantitative assay is based on the sandwich principal as previously described, utilizing the cell culture supernatants for the evaluation of IL-6 protein presence in LPS-treated cells in the presence of the evaluated samples.

For this protocol, RAW264.7 cells were treated with LPS prior to the measurement to stimulate IL-6 production and results are reported as pg/ml of IL-6 corresponding to the standard curve of IL-6 developed **(Table 4).** In this setting lower rates of IL-6 indicate higher anti-inflammatory potential.

Table 3: Anti-inflammatory potential of the samples expressed as IL-6 presence in LPS-stimulated RAW264.7 cells supernatants.

| Sample | Concentration | IL-6 (pg/ml) |
|---|---|---|
| S1 [NAR] | 25 $\mu$M | 720,000 |
| | 50 $\mu$M | 700,000 |
| | 100 $\mu$M | 455,000 |
| S2 [PLA[NAR]] | 25 $\mu$M | 609,000 |
| | 50 $\mu$M | 532,000 |
| | 100 $\mu$M | 296,000 |
| S3 CS[PLA[NAR]] | 25 $\mu$M | 250,000 |
| | 50 $\mu$M | 220,000 |
| | 100 $\mu$M | 200,000 |

Although concentration dependency was relevant for most samples, the encapsulated form of naringin in sample S3 presented remarkable and relatively stable reduction rate for IL-6, even in the lower concentrations.

**Example 6: In vitro evaluation of the cytotoxicity of the natural products and the prepared nanosystems**

**a) MTT cell viability in RAW264.7 cells**

The RAW 264.7 cells are monocyte/ macrophage-like cells. Macrophages cells have critical roles in the pathogenesis of numerous inflammatory disease processes by secreting various proinflammatory mediators, such as nitric oxide (NO), reactive oxygen species (ROS), cytokines, chemokines, and growth factors. Therefore, the modulation of macrophage-mediated inflammatory responses may be useful in the development of novel therapeutic approaches against these inflammatory diseases. (Kumar, P., Nagarajan, A., and Uchil, P.D. (2018). Analysis of Cell Viability by the MTT Assay. Cold Spring Harbor protocols 2018; Lee, J.Y., and Park, W. (2016). Anti-inflammatory effects of oroxylin A on RAW 264.7 mouse macrophages induced with polyinosinic-polycytidylic acid. Experimental and therapeutic medicine 12, 151-156.)

The MTT (3-[4,5-dimethylthiazol-2-yl]-2,5 diphenyl tetrazolium bromide) assay is based on the ability of viable cells with active metabolism to convert the yellow MTT to a purple formazan product, which determines mitochondrial activity. The MTT formazan product agglomerates as an insoluble residuum within the cells, which can be solubilized (in this protocol with DMSO) for homogeneous measurement. Thus, any increase or decrease in the number of viable cells can be detected by measuring the formazan concentration reflected in the optical density (OD) measurement at 540 nm with a reference absorption of 720 nm. This assay is broadly used to measure the in vitro cytotoxic effects of drugs on cell lines or primary cells.

For the experimental procedure a standard solution of MTT 1mg/mL in PBS was prepared which after filtration (0.22 mm filter (Millipore, Carrigtwohill, Ireland)) was stored in the dark (4-8 °C) until use. In brief, RAW 264.7 cells were seeded in a 96-well plate at a density of 30.000 cells/ in 100 $\mu$L of medium per well. After overnight incubation (37 °C, 5% CO2), the cells were treated with various concentrations (100, 50, 25 $\kappa\alpha\iota$ 10 $\mu$M, diluted in DMEM) of nanoparticles and pure compound (naringin). This was followed by incubation for 24 h, 48 h and 72 h. At the end of the incubation 10 $\mu$L of MTT was added to each well followed by incubation for 4 hours, for the formation of the formazan crystals. The formazan crystals were then dissolved by adding DMSO and stirring lightly for 30 min, after which the absorbance was measured in a plate reader at 540 nm with a reference absorbance of 720 nm.

All samples were tested in triplicate, against control (only cells), blank (no cells), and negative control (100, 50 and 25 $\mu$M standard silibinin solution was used as a "cell-killing" standard).

In this setting, the nanoparticles and pure naringin were evaluated in concentrations of 100$\mu$M, 50$\mu$M and 25$\mu$M, exhibiting the following potential after 24h, 48h and 72h of dilution of the nanosystems (Table 4).

Table 4: % cell viability after treatment of RAW264.7 cells with the samples

| Sample | Concentration | 24h | 48h | 72h |
|---|---|---|---|---|
| S1 [NAR] | 25 $\mu$M | 100 | NA | NA |
| | 50 $\mu$M | 100 | NA | NA |
| S2 [PLA[NAR]] | 25 $\mu$M | 100 | 100 | 100 |
| | 50 $\mu$M | 100 | 100 | 100 |
| S3 CS[PLA[NAR]] | 25 $\mu$M | 100 | 100 | 100 |
| | 50 $\mu$M | 100 | 100 | 100 |
| S4 CS[PLA[---]] | 25 $\mu$M | 100 | 100 | 100 |
| | 50 $\mu$M | 100 | 100 | 100 |
| PC (Silibinin) | 25 $\mu$M | 30 | NA | NA |
| | 50 $\mu$M | 20 | NA | NA |

In this setting none of the evaluated samples exhibited high levels of cytotoxicity. Pure Naringin (S1) and nanosystems (S2, S3, S4) resulted in full cell viability in the lower concentrations.

**Example 7: CCK-8 viability assay and morphological evaluation of primary human chondrocytes after incubation with the prepared nanosystems**

b) The viability of human primary chondrocytes was evaluated following incubation with increasing concentrations of either the pure Naringin (S1) or the nanosystems (Samples S2 and S3) at 2-10-50$\mu$M of encapsulated Naringin (1:5 serial dilutions) for 72h (higher concentrations were not used because the nanosystems become insoluble and aggregates sediment on the cell monolayer). For S2 this corresponded to 6,4 $\mu$g/ml (for 2 $\mu$M of Naringin), 32 $\mu$g/ml (for 10 $\mu$M of Naringin) and 158 $\mu$g/ml (for 50 $\mu$M of Naringin) respectively, of total NP concentration. For S3 (CS[PLA[NAR]]) the total NP concentrations to provide the same $\mu$M of encapsulated Naringin were 14,4 / 72 / 360 $\mu$g/m). As control for the nanosystems, a blank PLA nanoparticle preparation coated with chitosan (S4) was used, at the same $\mu$g/ml polymer concentration like the ones used to achieve the 2-10-50$\mu$M range for Naringin (the difference being that the blank contains no Naringin). Finally, the blank S4 was used at the 14,4/72/360 $\mu$g/ml concentration range (1:5 serial dilutions). Following incubation, the CCK-8 viability kit was used according to manufacturer's instructions (Sigma, 96992-500TESTS-F). Macroscopical and microscopical observation confirmed the proper complete solubility and dispersion of the Nanoparticles. Importantly, healthy cell morphology was observed (spindle-like attached cells with several processes) with concomitant absence of undissolved material.

Table 5 presents the mean relative viability results (CCK-8 assays from $\geq$3 independent repetitions of the experiment) normalized to the positive control which is set at 100 (in this case the positive control was treatment with 1$\mu$M Ibuprofen). Sample S3 consistently enhanced cell viability in a dose-dependent manner, with a boost to almost 150% at the 50$\mu$M final concentration of encapsulated naringin and outperformed most of the other nanosystems and the free Naringin.

Table 5. Effect of the natural products and the nanosystems on the relative cell viability of primary human chondrocytes. Measurements obtained using the CCK-8 viability kit. Values normalized to positive control (PC; set to 100; arbitrary unit). Samples' coding: S1= pure Naringin, S2=Naringin encapsulated in PLA nanoparticles [PLA[NAR]], S3=Naringin encapsulated in PLA nanoparticles coated with chitosan CS[PLA[NAR]], and S4=Blank PLA nanoparticles coated with chitosan, CS(PLA[---]).

| Cell viability (%) normalized to positive control (Ibuprofen) after treatment with the samples in primary human chondrocytes. | | |
|---|---|---|
| **Sample codes** | **Concentration** | **Mean % relative viability** |
| PC | 1 $\mu$M Ibuprofen | 100 |
| NC | 10 ng/ml TNF$\alpha$ | 118,41 |
| S1 [NAR] | 10 $\mu$M | 136.03 |
| | 50 $\mu$M | 130.12 |
| | 200 $\mu$M | 110.99 |
| S2 [PLA[NAR]] | 2 uM encapsulated Naringin | 139.58 |
| | 10 uM encapsulated Naringin | 93.63 |
| | 50 uM encapsulated Naringin | 85.43 |
| **S3 CS[PLA[NAR]]** | 2 uM encapsulated Naringin | 102,47 |
| | 10 uM encapsulated Naringin | 129,72 |
| | 50 uM encapsulated Naringin | **157.39** |
| S4 CS(PLA[---]] | Blank (no NAR): 14.4 $\mu$g/ml | 94.24 |
| | blank: 72 $\mu$g/ml | 106.26 |
| | blank: 360 $\mu$g/ml | 114,67 |

**Example 8: In vitro evaluation of the anti-catabolic activity of the natural products and the corresponding nanosystems**

**a) MMP-13 ELISA**

Matrix Mettaloproteinase 13 (MMP-13) is the main culprit in the destabilization of the anabolic/catabolic balance in the articular joint. MMP-13 tilts the balance towards catabolism and collagen II degradation which aggravates the OA pathology and is an enzyme that is secreted by chondrocytes in the extracellular environment. The aim was to measure whether treatment of cells with the nanosystems can lead to a detectable decrease in the MMP-13 levels, compared to TNF$\alpha$-treated cells that did not receive nanosystems. A decrease in MMP-13 levels is desirable, correlates with less degradation of cartilage and is widely used in the OA research field as a histopathological marker to evaluate amelioration of OA cellular pathology in the joint following an intervention. Finding Nanoparticles that can reduce MMP-13 levels in the assay is a promising lead for a favorable effect in a pre-clinical animal model *in vivo*. As Table 7 below shows, indeed the high concentration condition of S3 (CS[PLA[NAR]]) performed well and led to a detectable relative decrease (to 84,17%) in MMP-13 levels in the secretions of stimulated chondrocytes. Of note, the free Naringin (S1) did not give a detectable decrease in MMP-13 and this was considered an unexpected finding, meaning that the encapsulation of naringin in the PLA-coated with chitosan nanosystem seemed to work efficiently. The results in Table 6 are the mean relative values from at least 3 independent repetitions of the experiments.

Table 6. MMP-13 ELISA results as normalized values relative to negative control (NC, TNFα-stimulated cells without nanosystems) from 3 independent experiments. The standard deviations are omitted for simplicity. Samples' coding: S1=Naringin pure compound, S2=Naringin encapsulated in PLA nanoparticles [PLA[NAR]], S3=Naringin encapsulated in PLA nanoparticles coated with chitosan **CS[PLA[NAR]],** and S4=Blank PLA nanoparticles coated with chitosan, CS[PLA[---]].

| *MMP-13 secretion levels* *normalized to negative control (TNFα only) after co-treatment with the samples in primary human chondrocytes (ELISA detection).* | | |
| --- | --- | --- |
| **Sample codes** | **Concentration** | **Mean % relative MMP-13 secretion (72h)** |
| PC | 1 μM Ibuprofen | 55.40 |
| NC | 10 ng/ml TNFa | 100,00 |
| S1 [NAR] | 10 μM | 110,57 |
| | 50 μM | 113.28 |
| | 200 μM | 102,77 |
| S2 [PLA[NAR]] | 2 μM encapsulated Naringin | 136,67 |
| | 10 μM encapsulated Naringin | 116,33 |
| | 50 μM encapsulated Naringin | 109,67 |
| **S3 CS[PLA[NAR]]** | 2 μM encapsulated Naringin | 128,42 |
| | 10 μM encapsulated Naringin | 135,94 |
| | 50 μM encapsulated Naringin | **84,17** |
| S4 CS[PLA[---]] | blank: 14.4 μg/ml | 126,38 |
| | blank: 72 μg/ml | 109,67 |
| | blank: 360 μg/ml | 116.33 |

**Example 9: *In vivo* evaluation of the anti-inflammatory and antioxidant activity of the natural products and the corresponding nanosystems on an experimental animal model of osteoarthritis**

In order to understand the anti-inflammatory and antioxidant potential of the nanoparticles of the invention *in vivo* an experimental animal model of osteoarthritis (OA) was established. The OA lesions were caused by matrix degradation of articular cartilage using enzymatic means, such as collagenase type-II. Following the induction of osteoarthritis, the studied nanoparticles were administered for 8 weeks in which period the morphological and behavioral changes were measured and finally histological analysis of the articular cartilage samples concluded the *in vivo* study.

*Animals*

Healthy adult male Wistar rats (150-200 g) were used for the study, with water and food ad libitum, at a constant temperature of 22°C and controlled lighting (12 h light/12 h dark cycle). The animals were randomly assigned into 9 different groups of five animals per group (n=5): I) group I (Normal control) no collagenase injection, II) group II (OA-control) given collagenase injection but no treatment, III) group III given the pure compound naringin (10 mg/kg i.p), IV) group IV given the studied nanoparticle (sample 5:10 mg/kg i.a), V) group V given the blank nanoparticle (sample 6:10 mg/kg i.a), VI) group VI given the studied nanoparticle (sample 4:10mg/kg i.a) and VII) group VII given ibuprofen as reference standard (10 mg/kg i.p).

As far as the dispersion procedure of the injectables is concerned, all the natural compounds were dissolved in saline solution, whereas the studied nanoparticles were mechanically grinded in a mortar and pestle set with saline solution and Tween 20 (2% w/v) and heated at 36 °C before each treatment. There were six treatments administered every 10 days. At the end of the experiment rats were euthanized and both knees were collected for histological analysis.

*Induction* of OA

Rats were anesthetized with chloral hydrate. The right knee joints of Group I were injected with normal saline solution and served as a control. Animals from the groups II, III, IV, V, VI, VII were injected with collagenase type II (Clostridium histolyticum). Collagenase (125 collagen digestion unit/mg) was dissolved in saline, and it was injected intra-articularly into the right knee joint twice on days 1 and 4 of the experiment (Al-Saffar, F., H. Ganabadi and S. Fakurazi. Collagenase and sodium iodoacetate-induced experimental osteoarthritis model in Sprague dawley rats. Asian J. Sci. Res. 2: 1-13, 2009). The left knees of all animals were left untreated as a control.

**Results**

**Gross morphological observations**

Changes in body weight and knee diameter were measured on the 1st, 4th, 11th, 21st, 31st, 41st, 51st, 61st, 71st day before every injection. Knee diameter was measured using a digital caliper. At the onset of osteoarthritis redness and swelling of the joints and body weight loss is expected (Adães, S., Mendonça, M., Santos, T. N., Castro-Lopes, J. M., Ferreira-Gomes, J., & Neto, F. L. (2014). Intra-articular injection of collagenase in the knee of rats as an alternative model to study nociception associated with osteoarthritis. Arthritis Research & Therapy, 16(1), R10. doi:10.1186/ar4436; Kikuchi, T., Sakuta, T., & Yamaguchi, T. (1998). Intra-articular injection of collagenase induces experimental osteoarthritis in mature rabbits. Osteoarthritis and Cartilage, 6(3), 177-186. doi:10.1053/joca.1998.0110). A significant knee swelling progresses from the 2nd day, following the first collagenase injection, and after the second injection on the 4th day maximum swelling develops on the 5th day. A change in body weight is also measured as one of the parameters to assess the course of the disease in the OA animal models. Collagenase-injected animals present decreased body weights the first 10 days after injection. However, on administering the treatment injections (day 11th) the body weight once again increases. These changes were not observed in the saline group. (Tables 7-15)

Table 7: Changes in Body Weight (BW) g over treatment

| Sample | 1st injection CO/saline | 2nd injection CO/saline | 1st treatment | 2nd treatment | 3rd treatment | 4th treatment | 5th treatment | 6th treatment | final |
|---|---|---|---|---|---|---|---|---|---|
| Normal control | 220 | 220 | 235 | - | - | - | - | - | 235 |
| Arthritic control | 214 | 208 | 211 | - | - | - | - | - | 211 |
| S1 [NAR] | 247 | 236 | 239 | 247 | 276 | 294 | 317 | 326 | 333 |
| S3 CS[PLA[NAR]] | 239 | 232,6 | 244 | 253 | 258 | 252 | 276 | 294 | 304 |
| S4 CS[PLA[---]] | 222 | 213 | 229 | 240 | 273 | 277 | 251 | 290 | 303 |
| IBUPROFEN | 234 | 230 | 245 | 276 | 296 | 303 | 308 | 313 | 328 |

Table 8: % Changes in Body Weight (BW) g over treatment

| Sample | 11 days (before treatment) | 40 days (half of experimental protocol) | 70 days (end of experimental protocol) |
|---|---|---|---|
| Normal control | 6,8 | | |
| Arthritic Control | -1,4 | | |
| S1 [NAR] | -3,2 | 19,0 | 34,8 |
| S3 CS[PLA[NAR]] | 2,1 | 5,4 | 27,2 |

(continued)

| Sample | 11 days (before treatment) | 40 days (half of experimental protocol) | 70 days (end of experimental protocol) |
|---|---:|---:|---:|
| S4 CS[PLA[---]] | 3,2 | 24,8 | 36,5 |
| IBUPROFEN | 4,7 | 29,5 | 40,2 |

Table 9a: Changes in Right knee diameter (cm) over treatment

| Sample | 1st injection CO/saline | 2nd injection CO/saline | 1st treatment | 2nd treatment | 3rd treatment |
|---|---|---|---|---|---|
| Normal control | 1 | 1 | 1 | - | - |
| Arthritic control | 0,81 | 0,828 | 0,8412 | - | - |
| S1 [NAR] | 0,85 | 0,882 | 0,8852 | - | 0,9 |
| S3 CS[PLA[NAR]] | 0,805 | 0,812 | 0,8142 | 0, 8228 | 0,826 |
| S4 CS[PLA[---]] | 0,8072 | 0,8108 | 0,8116 | 0,8204 | 0,8222 |
| IBUPROFEN | 0,8128 | 0,8192 | 0,8202 | 0,81.8 | 0,8172 |

Table 9b: Changes in Right knee diameter (cm) over treatment

| Sample | 4th treatment | 5th treatment | 6th treatment | final |
|---|---|---|---|---|
| Normal control | - | - | - | 1 |
| Arthritic control | - | - | - | 0,8412 |
| S1 [NAR] | 0,8776 | 0,8712 | 0,87 | 0,87 |
| S3 CS[PLA[NAR]] | 0,811 | 0,8115 | 0,81025 | 0,81 |
| S4 CS[PLA[---]] | 0,8264 | 0,8254 | 0,8272 | 0,8322 |
| IBUPROFEN | 0,8174 | 0,817 | 0,816 | 0,8158 |

Table 10: % Changes in Right knee diameter (cm) over treatment

| Sample | 11 days (before treatment) | 40 days (half of experimental protocol) | 70 days (end of experimental protocol) |
|---|---|---|---|
| Normal control | 0 | - | - |
| Arthritic Control | 3,85 | - | - |
| S1 [NAR] | 4,14 | 3,25 | 2,35 |
| S3 CS[PLA[NAR]] | 1,14 | 0,75 | 0,62 |
| S4 CS[PLA[---]] | 0,55 | 2,38 | 3,10 |

(continued)

| Sample | 11 days (before treatment) | 40 days (half of experimental protocol) | 70 days (end of experimental protocol) |
|---|---|---|---|
| IBUPROFEN | 0,91 | 0,57 | 0,37 |

Table 11a: Changes in Left knee diameter (cm) over treatment

| Sample | 1st injection CO/saline | 2nd injection CO/saline | 1st treatment | 2nd treatment | 3rd treatment |
|---|---|---|---|---|---|
| Normal control | 1,033 | 1,033 | 1,033 | - | - |
| Arthritic control | 0,824 | 0,824 | 0,825 | - | - |
| S1 [NAR] | 0,8572 | 0,858 | 0,8594 | - | - |
| S3 CS[PLA[NAR]] | 0,81 | 0,81 | 0,81 | - | - |
| S4 CS[PLA[---]] | 0,8084 | 0,8085 | 0,809 | - | - |
| IBUPROFEN | 0,8158 | 0,816 | 0,816 | - | - |

Table 11b: Changes in Left knee diameter (cm) over treatment

| Sample | 4th treatment | 5th treatment | 6th treatment | final |
|---|---|---|---|---|
| Normal control | - | - | - | 1,033 |
| Arthritic control | - | - | - | 0,825 |
| S1 [NAR] | 0,864 | - | - | 0,8644 |
| S3 CS[PLA[NAR]] | 0,8112 | - | - | 0,8132 |
| S4 CS[PLA[---]] | 0,0,8112 | - | - | 0,8132 |
| IBUPROFEN | 0,8184 | - | - | 0,8194 |

Table 12: % Changes in Left knee diameter (cm) over treatment

| Sample | 11 days (before treatment) | 40 days (half of experimental protocol) | 70 days (end of experimental protocol) |
|---|---|---|---|
| Normal control | 0 | - | - |
| Arthritic Control | 0,12 | - | - |
| S1 [NAR] | 0,26 | 0,79 | 0,84 |
| S3 CS[PLA[NAR]] | 0,00 | 0,15 | 0,40 |
| S4 CS[PLA[---]] | 0, 07 | 0,35 | 0,59 |
| IBUPROFEN | 0,02 | 0,32 | 0,44 |

**Behavioral testing**

For the evaluation of the effectiveness and time of release of the encapsulated compounds, all animals were subjected to a series of behavioral testing in addition to the measurements of body weight and knees diameter change. The experimental protocol of the present study followed the combination of three different behavioral tests, which are von Frey mechanical allodynia test, grid walking and slope walking test. Behavioral tests are performed in three phases, at the beginning before the start of treatment, in the middle of treatment (approximately 35-40 days) and at the end (after the 70th day).

**A. Assessment of Pain-Related Behavior**

Mechanical allodynia was tested by the von Frey method which detects pain threshold as the paw withdrawal response. For the testing, rats were placed into plastic chambers that allow access to the plantar surface of the hind paws. The electronic von Frey filament was then applied with gradually increased pressure to the plantar surface of both the right collagenase-injected and the left control paw until a paw withdrawal response was observed. Rats after OA induction showed a marked decrease of hind paw withdrawal threshold, denoting the presence of tactile allodynia. On the commencement of treatment with the studied nanoparticles (Sample S3) and pure compound naringin (Sample S1), paw withdrawal thresholds of the treated animals presented an increase. Such results were also observed in the positive control group treated with the drug ibuprofen, as anticipated.

In Table 15 below, the nociceptive threshold is defined as:

$$\text{nociceptive threshold (\%)} = \text{(nociceptive threshold on right paw)}/\text{((nociceptive threshold on right paw + nociceptive threshold of left paw))} *100$$

Table 13: von Frey-paw withdrawal threshold (g)

| Sample | left-control paw | 11 days (before treatment) | 40 days (half of experimental protocol) | 70 days (end of experimental protocol) |
|---|---|---|---|---|
| Normal control | 86,77 | 86,00 | 87,93 | - |
| Arthritic control | 86,6 | 62,4 | 64,36 | - |
| S1[NAR] | 88,68 | 63 | 81,12 | 83,96 |
| S3 CS[PLA [NAR]] | 93,24 | 63,68 | 70,94 | 76,9 |
| S4 CS[PLA [---]] | 85,96 | 62, 8 | - | 65 |
| IBU | 93,9 | 59,78 | - | 83,8 |

Table 14: Paw Withdrawal Threshold (%)

| Sample | 11 days (before treatment) | 40 days (half of experimental protocol) | 70 days (end of experimental protocol) |
|---|---|---|---|
| Normal control | 49,78 | 50,05 | - |
| Arthritic control | 41,88 | 41,80 | - |
| S1[NAR] | 41,53 | 45,55 | 45,95 |
| S3 CS[PLA [NAR]] | 40,58 | 42,87 | 44,75 |

(continued)

| Sample | 11 days (before treatment) | 40 days (half of experimental protocol) | 70 days (end of experimental protocol) |
|---|---|---|---|
| S4 CS[PLA[---]] | 42,22 | - | 42,10 |
| IBU | 38,90 | - | 45,44 |

**B. Grid-Walking Test**

In this test the rats were allowed to explore freely for 4 minutes in a wire grid and when one of the limbs fell below the grid surface during free walking, the observer counted the number of foot-fault errors (footslips) (Starkey, M. L., Barritt, A. W., Yip, P. K., Davies, M., Hamers, F. P. T., McMahon, S. B., & Bradbury, E. J. (2005). Assessing behaviour function following a pyramidotomy lesion of the corticospinal tract in adult mice. Experimental Neurology, 195(2), 524-539. doi:10.1016/j.expneurol.2005.0).

Table 15: Foot fault in grid-walk test (4 min)

| Sample | 11 days left paw | 11 days right paw | 40 days left paw | 40 days right paw | 70 days left paw | 70 days right paw |
|---|---|---|---|---|---|---|
| Normal control | 1,3 | 1,3 | 1,3 | 1 | - | - |
| Arthritic control | 5,8 | 7,6 | 1,2 | 3,6 | - | - |
| S1 [NAR] | 3,4 | 5,6 | 2 | 3 | 2,8 | 2,4 |
| S3 CS[PLA[NAR]] | 2 | 4 | 0,6 | 1,2 | 0,8 | 1,8 |
| S4 CS[PLA[---]] | 1,2 | 2,6 | 1,8 | 2,6 | 0,4 | 2 |
| IBUPROFEN | 1,4 | 2,8 | 1,8 | 1,2 | 1 | 1 |

**C. Slope Walking**

Walking on an inclined plane was used for the clinical assessment of motor function in rats after induction of OA. The method consists of placing the animal on an inclined plane and then measuring the time (seconds) needed for the animals to go up the walking tray.

Table 16: Slope Walking (sec)

| Sample | 11 days (before treatment) | 40 days (half of experimental protocol) | 70 days (end of experimental protocol |
|---|---|---|---|
| Normal control | 9,3 | 9,3 | - |
| Arthritic control | 10 | 10,2 | - |
| S1 [NAR] | 10,3 | 8,9 | 8,6 |
| S3 CS[PLA[NAR]] | 10,4 | 9,5 | 9,3 |
| S4 CS[PLA[---]] | 9,6 | 9,7 | 10 |
| IBUPROFEN | 9,8 | 9,2 | 8,9 |

The results of macroscopic and behavioral evaluation of the animals treated with the nanosystems of the invention (Sample S3), suggest that there is a slow release of the encapsulated compound at the inflammation site that ameliorates the knee diameter increase and joint pain, compared to the blank nanoparticles (Sample S4). As intra-articular injection can induce acute phlogistic/inflammatory severe reactions, in this study the exposure to nanoparticles did not lead to deleterious reactions for the animals. However, the intra-articular administration of the nanoparticles of the invention opposed to the intraperitoneal administration of the natural compound (naringin) and positive reference drug (ibuprofen) seem to induce slight and reversible inflammatory responses in the OA- induced right knees. The *in vivo* studies show that the encapsulation of naringin in the nanoparticles of the invention have beneficial effect on the progression of OA.

**Claims**

1. A nanoparticle comprising a therapeutic agent exhibiting anti-inflammatory or analgesic activity encapsulated in polylactic acid (PLA) or poly (lactic-co-glycolic) acid (PLGA), wherein the nanoparticle is coated with chitosan.

2. The nanoparticle according to claim 1, wherein the therapeutic agent is selected from naringin, cannabidiol, hydroxytyrosol, rosmarinic acid, baicalein, chrysin, quercetine, silymarin, cyanidin-3-O-glucoside, peonidin-3-O-glucoside.

3. The nanoparticle according to claim 1 or 2, wherein the therapeutic agent is naringin.

4. The nanoparticle according to any one of the preceding claims, wherein PLA has an average molecular weight from 10000g/mol to 70000g/mol

5. The nanoparticle according to any one of the preceding claims, wherein PLGA has an average molecular weight from 5000g/mol to 40000g/mol.

6. The nanoparticle according to any one of the preceding claims, wherein the molar ratio of lactic acid to glycolic acid in PLGA is from 45:50 to 75:25.

7. The nanoparticle according to any one of the preceding claims, wherein the average molecular weight of chitosan is from 5000 g/mol to 60000g/mol.

8. The nanoparticle according to any one of the preceding claims, wherein the content of PLA or of PLGA in the nanoparticle is from 20%w/w to 35%w/w.

9. The nanoparticle according to any one of the preceding claims, wherein the content of chitosan in the nanoparticle is from 55 %w/w to 75 %w/w.

10. The nanoparticle according to any one of the preceding claims, wherein the content of the therapeutic agent in the nanoparticle is from 1% w/w to 10%w/w.

11. The nanoparticle according to any one of the preceding claims, wherein the mass ratio of PLA to chitosan, or the mass ratio of PLGA to chitosan in the nanoparticle is from 1:2 to 1:3.

12. The nanoparticle according to any one of the preceding claims, wherein the nanoparticle further comprises an emulsifier or a crosslinker.

13. The nanoparticle according to any one of the preceding claims, for use in the treatment of osteoarthritis.

14. A pharmaceutical composition comprising a nanoparticle as defined in any one of claims 1 to 13 and a pharmaceutically acceptable carrier.

15. The pharmaceutical composition according to claim 14 for use in the treatment of osteoarthritis.

Europäisches
Patentamt

European
Patent Office

Office européen
des brevets

**EUROPEAN SEARCH REPORT**

Application Number

EP 22 38 6094

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ALSHEMEMRY ABDULLAH ET AL: "Chitosan-coated poly (lactic-co-glycolide) nanoparticles for dual delivery of doxorubicin and naringin against MCF-7 cells", JOURNAL OF DRUG DELIVERY SCIENCE AND TECHNOLOGY, vol. 68, 27 December 2021 (2021-12-27), pages 103036-13, XP93041518, FR ISSN: 1773-2247, DOI: 10.1016/j.jddst.2021.103036 | 1-3,5,6, 10,14 | INV. A61K9/00 A61K9/51 |
| Y | * page 2; table 1 * * items 2.2.1. Formulation development, 2.2.2. Freeze-drying of drug-loaded uncoated- and CS-coated NPs; page 3 * | 7-9,11 | |
| X | ARAFA MONA G ET AL: "Chitosan-Coated PLGA Nanoparticles for Enhanced Ocular Anti-Inflammatory Efficacy of Atorvastatin Calcium", INTERNATIONAL JOURNAL OF NANOMEDICINE, vol. Volume 15, 28 February 2020 (2020-02-28), pages 1335-1347, XP093041482, DOI: 10.2147/IJN.S237314 Retrieved from the Internet: URL:https://www.ncbi.nlm.nih.gov/pmc/artic les/PMC7053815/pdf/ijn-15-1335.pdf> | 1,6 | TECHNICAL FIELDS SEARCHED (IPC) A61K |
| Y | * items Materials and Reagents, Preparation of AT-PLGA-CS-NPs; page 1336 * | 7-9,11 | |

-----

-/--

The present search report has been drawn up for all claims

| Place of search | Date of completion of the search | Examiner |
|---|---|---|
| The Hague | 10 May 2023 | Raposo, Antonio |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

                                                                                                                                                                                                           
& : member of the same patent family, corresponding
   document

EPO FORM 1503 03.82 (P04C01)

page 1 of 2

**EUROPEAN SEARCH REPORT**

Application Number

EP 22 38 6094

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KHANAL SHALIL ET AL: "pH-Responsive PLGA Nanoparticle for Controlled Payload Delivery of Diclofenac Sodium", JOURNAL OF FUNCTIONAL BIOMATERIALS, vol. 7, no. 3, 1 August 2016 (2016-08-01), pages 1-12, XP93041505, DOI: 10.3390/jfb7030021 Retrieved from the Internet: URL:https://www.ncbi.nlm.nih.gov/pmc/articles/PMC5040994/pdf/jfb-07-00021.pdf> | 1,6 | |
| Y | * item Design and Characterization of PLGA Nanoparticles; page 3 * * items 3.1. Materials, 3.2. Nanoparticle Preparation; pages 7-8 * | 7-9,11 | |
| X | SALAMA ALAA H. ET AL: "Etoricoxib-loaded bio-adhesive hybridized polylactic acid-based nanoparticles as an intra-articular injection for the treatment of osteoarthritis", INTERNATIONAL JOURNAL OF PHARMACEUTICS, vol. 11, 7 February 2020 (2020-02-07), page 1, XP93041693, NL ISSN: 0378-5173, DOI: 10.1016/j.ijpharm.2020.119081 | 1,4, 12-15 | TECHNICAL FIELDS SEARCHED (IPC) |
| Y | * the whole document * | 7-9,11 | |

The present search report has been drawn up for all claims

| Place of search | Date of completion of the search | Examiner |
|---|---|---|
| The Hague | 10 May 2023 | Raposo, Antonio |

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **ALI MOBASHERI ; MARK BATT.** An update on the pathophysiology of osteoarthritis. *Annals of Physical and Rehabilitation Medicine,* 2016, vol. 59 (5-6), ISSN 1877-0657, 333-339, https://doi.org/10.1016/j.rehab.2016.07.004
- United States Bone and Joint Initiative: The Burden of Musculoskeletal Diseases. United States (BMUS), 25 February 2020
- **LOTZ M ; MARTEL-PELLETIER J ; CHRISTIANSEN C et al.** Value of biomarkers in osteoarthritis: current status and perspectives [published correction appears. *in Ann Rheum Dis.,* 25 May 2017
- *Ann Rheum Dis.,* 2013, vol. 72 (11), 1756-1763
- *Annals of Physical and Rehabilitation Medicine,* 2016, vol. 59 (5-6), ISSN 1877-0657, 333-339, hftps://doi.org/10.1016/j.rehab.2016.07.004
- **FLORES-GARZA, P.P. ; GARCIA-ESPINOZA, Ó.A. ; SALAS-LONGORIA, K. ; SALAS-FRAIRE, Ó.** Association between ischiotibial muscle flexibility, functional capacity and pain in patients with knee osteoarthritis. *Med. Univ.,* 2017, vol. 19, 111-114
- **ARUL PRAGASSAME S ; MOHANDAS KURUP VK ; SOUNDARYA N.** A comparative study on the effectiveness of PNF stretching versus static stretching on Pain and Hamstring flexibility in osteoarthritis knee patients. *Int. J. Res. Pharm. Sci.,* 2019
- Osteoarthritis. **HERNÁNDEZ-DÍAZ, C. ; VAN SCHOOR, N. ; KHALIL, A.A.F.** Comorbidity in Rheumatic Diseases. Springer Science and Business Media, 2017, 197-206
- **WALLIS, J.A. ; BARTON, C.J. ; BRUSCO, N.K. ; KEMP, J.L. ; SHERWOOD, J. ; YOUNG, K. ; JENNINGS, S. ; TRIVETT, A. ; ACKERMAN, I.N.** Exploring views of orthopaedic surgeons, rheumatologists and general practitioners about osteoarthritis management. *Musculoskeletal Care,* 2021
- **JADIDI, S.** A Review of Non-Surgical Pain Management in Osteoarthritis. *Cureus,* 2020
- **KAN, H.S. ; CHAN, P.K. ; CHIU, K.Y. ; YAN, C.H. ; YEUNG, S.S. ; NG, Y.L. ; SHIU, K.W. ; HO, T.** Non-surgical treatment of knee osteoarthritis. *Hong Kong Med. J.,* 2019

- **NELSON, A.E. ; ALLEN, K.D. ; GOLIGHTLY, Y.M. ; GOODE, A.P. ; JORDAN, J.M.** A systematic review of recommendations and guidelines for the management of osteoarthritis: The Chronic Osteoarthritis Management Initiative of the U.S. Bone and Joint Initiative. *Semin. Arthritis Rheum.,* 2014
- **CROFFORD, L.J.** Use of NSAIDs in treating patients with arthritis. *Arthritis Res. Ther.,* 2013
- **VAN DE LAAR, M.** Pain Treatment in Arthritis-Related Pain: Beyond NSAIDs. *Open Rheumatol. J.,* 2012
- **DELIGIANNIDOU, G.E. ; PAPADOPOULOS, R.E. ; KONTOGIORGIS, C. ; DETSI, A. ; BEZIRTZOGLOU, E. ; CONSTANTINIDES, T.** Unraveling natural products' role in osteoarthritis management-an overview. *Antioxidants,* 2020
- **KUMAR, P. ; NAGARAJAN, A. ; UCHIL, P.D.** Analysis of Cell Viability by the MTT Assay. *Cold Spring Harbor protocols,* 2018
- **LEE, J.Y. ; PARK, W.** Anti-inflammatory effects of oroxylin A on RAW 264.7 mouse macrophages induced with polyinosinic-polycytidylic acid. *Experimental and therapeutic medicine,* 2016, vol. 12, 151-156
- **AL-SAFFAR, F. ; H. GANABADI ; S. FAKURAZI.** Collagenase and sodium iodoacetate-induced experimental osteoarthritis model in Sprague dawley rats. *Asian J. Sci. Res.,* 2009, vol. 2, 1-13
- **ADÃES, S. ; MENDONÇA, M. ; SANTOS, T. N. ; CASTRO-LOPES, J. M. ; FERREIRA-GOMES, J. ; NETO, F. L.** Intra-articular injection of collagenase in the knee of rats as an alternative model to study nociception associated with osteoarthritis. *Arthritis Research & Therapy,* 2014, vol. 16 (1), R10
- **KIKUCHI, T. ; SAKUTA, T. ; YAMAGUCHI, T.** Intra-articular injection of collagenase induces experimental osteoarthritis in mature rabbits. *Osteoarthritis and Cartilage,* 1998, vol. 6 (3), 177-186
- **STARKEY, M. L. ; BARRITT, A. W. ; YIP, P. K. ; DAVIES, M. ; HAMERS, F. P. T. ; MCMAHON, S. B. ; BRADBURY, E. J.** Assessing behavioural function following a pyramidotomy lesion of the corticospinal tract in adult mice. *Experimental Neurology,* 2005, vol. 195 (2), 524-539